# EUROPEAN PATENT SPECIFICATION

(11) **EP 1 202 062 B1**
(45) Date of publication and mention of the grant of the patent: **28.02.2007**
(21) Application number: 00123706.4
(22) Date of filing: 31.10.2000
(51) Int. Cl.: G01N 33/545, C12Q 1/68

(54) **Patterned surfaces for bioconjugation and their preparation**
Gemusterte Polymeroberflächen geeignet für Biokonjugationen und Verfahren zu ihrer Herstellung
Surfaces polymères à motifs pour la bioconjugaison et leur procédé de préparation

(43) Date of publication of application: 02.05.2002
(73) Proprietor: Micronas GmbH, 79108 Freiburg i. Br. (DE)
(72) Inventor: Klapproth, Holger, 79108 Freiburg (DE); Wagner, Gerhard, 79183 Waldkirch (DE)
(74) Representative: Göhring, Robert

(56) References cited:
- EP-A- 0 301 141
- WO-A-00/12575
- WO-A-00/43539
- WO-A-97/14963
- US-A- 5 262 297
- US-A- 5 466 348
- US-A- 5 695 925

## Description

Due to the steadily growing importance of microtechniques in a wide variety of scientific applications, the development of systems which allow the interaction of molecules with surfaces remains a critical issue. Such interactions include the possibility of removing specific molecules from a sample, e.g. to facilitate their analysis/detection, but also of presenting molecules on a surface, thus allowing subsequent reactions to take place. These principles for the immobilization of molecules can be applied in sensor or chromatographic systems or for the provision of modified surfaces in general.

In recent years there have been numerous approaches to fabricate sensor chips which are based on self-assembled monolayers (SAM's) of bifunctional molecules (so-called linkers) which directly or indirectly couple sample molecules to the sensor surface. Typically, these bifunctional molecules or linkers carry a silane or thiol/disulfide moiety in order to achieve a bond with the inorganic surface and an additional functional group (e.g. amino or epoxide groups) which interacts with sample molecules, often contained in biological samples in the form of an oligonucleotide, a protein or a polysaccharide etc. (cf. for example Chrisey, Lee and O'Ferrell in Nucleic Acids Research 1996, Vol. 24, pp. 3031-3039; Gray, Case-Green, Fell, Dobson and Southern in Langmuir 1997, Vol. 13, pp. 2833-2842 and the further references cited therein). For technical reasons these sensor chips of the prior art must have planar surfaces for pattering.

While the formation of a direct bond between the bifunctional compound and the sample molecule is possible, the sample molecules do not necessarily interact directly with the couplers forming the monolayer. Alternatively, appropriate immobilized biomolecules themselves can act as probes for the detection of sample molecules. Such probe molecules can equally be immobilized via a reaction with the free functional groups of the monolayer. In particular, if biomolecules are used as probe molecules, their presence may significantly enhance the specificity of the interaction of the sample molecules with the modified surface. For example, in cases where the fast analysis of a sample of DNA fragments or molecules is required, the monolayers of bifunctional molecules can first be brought in contact with synthetic oligonucleotides which will thus be immobilized. Subsequently, the hybridization of specific molecules, such as compatible strands from a sample is detected, e.g. via fluorescence microscopy, if dye-labeled sample molecules are used.

Although these techniques are well established for this purpose, the application of standard detection methods is problematic, especially in cases where the surface area available for the detection of one specific type of sample molecules is restricted, e.g. if a variety of molecules is to be analyzed in a parallel process, since the monolayers are limited in their graft density. For example, since the number of hybridized double strands per surface unit of a sensor can not easily be increased, suitable detectors have to meet very high requirements with regard to their sensitivity. Thus, the minimum surface area on a sensor necessary for the detection of one type of oligonucleotide can not be easily reduced.

Moreover, the maximum density, i.e. one sample or probe molecule per functional group of the couplers can hardly be attained, since due to sterical hindrance on the two-dimensionally extended monolayer, only a fraction of the functional groups will be able to react with sample or probe molecules. Thus, the overall graft density is low and normally not well defined.

Similar problems with regard to the limited number of reaction sites per surface unit can arise in other applications, where it is desirable to immobilize an increased amount of molecules on a surface.

Various attempts have been made to overcome the problems outlined above. As regards the analysis of oligonucleotides, it has been tried to increase the graft density on the surface by using oligomers or polymers which carry an oligonucleotide strand (or a functional group for its attachment) together with a suitable group which allows the bonding of these oligomers or polymers to the surface of the sensor chip. Due to the increased flexibility of the oligomeric or polymeric chains, a larger fraction of the bifunctional oligomer or polymer molecules which are coupled to the surface is able to immobilize oligonucleotide probe molecules.

However, the total oligonucleotide graft density is not significantly increased, because the graft density of the bifunctional oligomeric or polymeric molecules on the surface is limited. This is a consequence of the fact that the self-assembly of the oligomers or polymers is hindered for kinetic reasons, because once the sensor surface is covered with such molecules, further polymers will have to diffuse against a concentration gradient in order to reach the surface.

A different approach to the above-mentioned self-assembled monolayers of bifunctional molecules for immobilization is using networks for DNA analysis. A disadvantage is that these networks are not coupled to the sensor surface and are not structured, i.e. do not form patterned arrays. Moreover, since the networks must be swellable in the used hybridization medium there is a risk that the network is detached from the surface.

A further general disadvantage or problem of the prior art approaches is that the range of suitable surfaces is limited to those surfaces for which covalently binding bifunctional linker systems are known.

Moreover, since pattering active surfaces for bioconjugation is often performed by print techniques, only substrates having a planar surface may be used. For non-planar surfaces it is even with ink-jet printing not satisfactorily possible to obtain an accurate and reproducible pattern, in particular if a high spatial resolution is desired or necessary.

In addition, scaling up the production of patterned active surfaces for bioconjugation is not possible without further ado. The various steps to be performed require for automatic high throughput production more sophisticated apparatuses or production lines. In fact, patterned active surfaces for bioconjugation are for practical reasons up to now produced batchwise with all problems regarding reproducibility between the batches.

Accordingly, it is an object of the present invention to provide a method allowing the large scale production of patterned active surfaces for bioconjugation, wherein the number of molecules interacting per surface unit is markedly increased compared to conventional monolayers of bifunctional molecules, and wherein the density of available interaction sites is higher than that obtained from the reaction of bifunctional polymers or oligomers with the surface, and which method is not limited to any particular surface material or shape.

The invention thus relates to a method for the large scale production of patterned active surface for bioconjugation comprising the steps of:
(a) preparing a self-supporting film of a polyfunctional polymer network comprising an assembly of cross-linked polymer subchains, wherein each polymer subchain comprises a multitude of identical of different repeating units carrying one or more functional groups which allow an interaction of the polymer with one or more probe molecules,
(b) providing said self-supporting film with patterned arrays of said one or more probe molecules via an interaction with said functional groups,
(c) fixing said self-supporting film on a solid surface,
(d) wherein said functional groups of said polyfunctional polymer network are selected from the group consisting of carboxylic acids, maleinimides, N-hydroxy succinimides, epoxides, isothiocyanates, isocyanates, azides, NHS ester, glycidylester, acrylic or methacrylic acid N-hydroxysuccinimies, N-methacryloyl-6-aminopropanoic acid hydroxysuccinimide ester, N-methacryloyl-6-aminocapronic acid hydroxysuccinimide ester or acrylic or methacryl acid glycidyl esters.

Advantageously and thus preferred the polymer subchains of the polyfunctional polymer network comprise segments that make said polymer network water-swellable, for example said water-swellability is provided by monomers selected from the group consisting of acrylic acid, methacrylic acid, dimethyl acrylamide and vinyl pyrrolidone.

For preparing the cross-linked polymer subchains of the polyfunctional polymer network for example bisacrylates, bismethacrylates or bisacrylamides may be used as an initiator. It should be noted that there are no special limitations regarding the cross-linker and that any conventional cross-linker may be used.

The functional groups of the polyfunctional polymer network for example may be selected from carboxylic acids, maleinimides, N-hydroxy succinimides, epoxides, isothiocyanates, isocyanates or azides.

Advantageously and thus preferred each of the probe molecules interacting with the functional groups of the polyfunctional polymer network may be a partner of a specifically interacting system of complementary binding partners.

For example said specifically interacting system of complementary binding partners may be based on nucleic acid/complementary nucleic acid, peptide nucleic acid/nucleic acid, enzyme/substrate, receptor/effector, lectin/sugar, antibody/antigen, avidin/biotin or streptavidin/biotin interaction. It should be appreciated that the "meshes" of the polyfunctional polymer network are wide enough to allow an unrestricted access of the respective complementary binding partners. This unrestricted access is also supported by the water-swellability of the network.

In step (c) of the inventive method as defined above fixing the self-supporting film onto the solid surface for example may be performed by using any conventional reactive glue or any conventional bifunctional linker system which comprises one or more functional groups suitable for covalently binding said linker system to said solid surface and one ore more functional groups for covalently binding said polyfunctional polymer network to said covalently bound linker system.

A suitable reactive glue is for example a conventional acrylic adhesive. It should be noted in this context that there are no particular limitations regarding the glue subject to the fixed self-supporting film not being detached from the solid surface under the given reaction conditions, e.g. for hybridization of probe and sample oligonucleotides.

A suitable bifunctional linker system for example comprises a halogen silane, an alkoxy silane, an acyloxy silane, an amino silane, a disulphide or a thiol group for covalently binding the linker system to a solid surface.

Further, the bifunctional linker system comprises a group for covalently binding the polyfunctional polymer network to the covalently bound linker system, for example a photoreactive group such as groups consisting of or containing aromatic ketones or aromatic ketones containing sulphur, e.g. aromatic β-keto sulfides, aromatic β-keto sulfoxides or aromatic β-keto sulfones.

Specific examples for the photoreactive group are an anthrathione group or a derivative thereof, an anthraquinone group or a derivative thereof, a benzophenone group or a derivative thereof. A benzophenone group is preferred.

The solid surface for fixing the self-supporting film is not particularly limited and may be made of any material. Examples are a metal or semimetal surface, a metal oxide or semimetal oxide surface, or a polymer surface. Moreover, the surface may have any dimensions and shapes and is not limited to a specific surface geometry such as planar surfaces. For example, the surface may be planar or non-planar, e.g. convex or concave, and even prisms or spheres may be used.

In another embodiment of the inventive method the self-supporting film of a polyfunctional polymer network is in step (a) formed on one surface of a carrier film and in step (c) said carrier film is fixed on the solid surface with the other surface. There are no limitations with respect to the carrier film, i.e. any conventional polymer film may be used, and it should be noted that the use of a carrier film is absolutely optional. The mechanical strength of the self-supporting film is sufficiently high to allow a convenient handling, for example for transferring the film onto the solid surface for fixing.

In a preferred embodiment of the inventive method the self-supporting film, optionally on a carrier film, is in a further step following step (b) cut into sheets or an endless tape of a desired format and may be stably stored in this form and in any amount until use. The tape may preferably further optionally be wind-up onto a drum.

A special advantage of such an "endless chip" in tape form on a drum is that with a simple apparatus the "endless chip" may automatically and with high throughput transferred and fixed to substrates providing the solid surface. For example, the substrates in a given format, e.g. glass microscope slides, and covered with a layer of an adhesive or, for example, a photoreactive bifunctional linker system may be supplied to the apparatus by means of a conveyor belt or an equivalent transporting means. In the apparatus the "endless chip" is unwind, cut into an appropriate length and fixed to the surface, for example by using a suitable feed roll and applying heat or ultraviolet irradiation. Suitable production lines for this purpose are either known in the art, for example for laminating a foil to a book cover, or may easily be adapted for conducting the inventive method. A particular advantage of the "endless chip" is that it may easily be adapted by the end user (by simply cutting it into the correct shape) to any desired or given chip reader geometry.

In the following the present invention is explained in more detail and for illustration only. The examples are not to be construed as any limitation of the scope of the invention as defined in the appending claims.

The term "interaction", as used in this specification, includes the formation of covalent bonds, as well as attractive ionic and van-der-Waal's forces and hydrogen bonds. The respective functional moiety within the polyfunctional polymer network or the probe molecules, which defines the type of interaction, will be selected according to the desired application of the patterned active surface for bioconjugation to be provided.

The expression "immobilize" is used hereinafter for an interaction of molecules with the polyfunctional polymer network resulting in the formation of a bond which is permanent under the chosen conditions. For example, probe molecules are immobilized by the polyfunctional network during their application on a sensor surface. However, by changing conditions (e.g. pH-value, addition of specific cleaving agents) an immobilization may sometimes be reversed.

The term "sample molecule" shall be used herein for molecules which are present in a sample and which couple temporarily or permanently to the polyfunctional network. There are two general principles for an interaction of the polyfunctional network with the sample molecules. In a first embodiment, the functional groups comprised within the polyfunctional network are chosen in order to allow a direct interaction of the chains with the sample molecules. In a second embodiment, probe molecules are immobilized at the functional groups of the polyfunctional network, and an interaction takes place between those probe molecules and the sample molecules.

Suitable probe molecules are molecules which are at least bifunctional, so that after their coupling to the polyfunctional polymer network new interaction sites are present in the polyfunctional network which allow an interaction with sample molecules. Preferably, the probe molecules provide highly specific interaction sites for the sample molecules. They can be derived from natural or non-natural sources. Particularly preferred probe molecules are biomolecules such as nucleic acids, including DNA, RNA or PNA (peptide nucleic acid), most preferably oligonucleotides or aptamers, polysaccharides, proteins including glycosidically modified proteins or antibodies, enzymes, cytokines, chemokines, peptide hormones or antibiotics, and peptides. In order to ensure a sufficient stability, e.g. during a sensor application, the probe molecules are preferably covalently bound to the polyfunctional polymer network. It should be noted that the probe molecules may be the same or different. In the latter case the parallel detection of a plurality of sample molecules is possible.

The introduction of branched polymers into the network is possible, if desired. In some cases addition of comonomers which allow a tailoring of the physical properties of the network depending on the desired application is appropriate.

The minimum components of the network are the functionalized repeating units and the cross-linking units. For the functionalized repeating units the subchains of the polymer network contain repeating units which carry at least one of the functional groups which can interact with sample or probe molecules. However, in order to impart certain advantageous properties to the polyfunctional polymer network, a copolymer, formed from these monomers with specific functional groups for the interaction with sample or probe molecules (hereinafter referred to as "functionalized monomers") together with other comonomers can be used.

For example, the reaction of the sample or probe molecules with the polyfunctional polymer network is significantly facilitated if the polyfunctional polymer network is swellable in the solvent containing these molecules, so that comonomers should preferably be chosen which show a strong interaction with the solvent in question. This can be achieved by using comonomers which improve the swellability of the network. Since, in a most preferred embodiment of the present invention, biomolecules, which are normally present in aqueous solutions, interact with the polyfunctional polymer network, said polyfunctional polymer network is preferably water-swellable.

Thus, for example, one or more comonomers can be used which are polar, or even soluble in water, if a homopolymer of functionalized monomers does not show sufficient interaction with water to allow a fast reaction of the molecules to be detected with the functional groups. Both types of monomers, functionalized as well as comonomers, preferably contain a C-C double bond which can react in a radical polymerization reaction. Examples for suitable comonomers which yield a water swellable polymer are acrylic acid, methacrylic acid and derivatives thereof, e.g. esters and amides of these acids with alcohols or amines preferably comprising 1 to 12 carbon atoms.

Common examples of this group of monomers are hydroxyethyl methacrylate, acrylamide and dimethyl acrylamide. Another suitable monomer is vinyl pyrrolidone. It is also possible to use monomers that yield at first water insoluble polymers which can then be transferred to water soluble derivatives. A suitable example for this group of polymers is polyvinyl alcohol which can be obtained, for example, by saponification of polyvinyl acetate.

If a copolymer is used, the ratio of comonomers to functionalized monomers is determined prior to the polymerization process in order to define the composition of the resulting polymer chains of the polyfunctional polymer network. Preferably, the ratio of the comonomers to the functionalized monomers ranges from 50/1 to 1/1, more preferably form 20/1 to 2/1.

The functional groups which are necessary to allow an interaction of the polyfunctional polymer network with the sample or probe molecules are preferably present in side chains of the polymer subchains of the polyfunctional polymer network. A "multitude" of functional groups comprised in polymer subchains of the polyfunctional polymer network of the present invention means at least two, but preferably more than two groups per polymer subchain. Since the concerned functional groups are preferably comprised in repeating units forming the polymer subchains of the polyfunctional polymer network, their number may amount up to several thousand, e.g. up to 10000 of these groups present in a single subchain, depending on the size of the probe or sample molecule to be immobilized. Preferably, each chain comprises 20 to 1000 of these functional groups.

Suitable functionalized repeating units which are present in the polymer subchains of the polyfunctional polymer network are those repeating units which comprise a polymerizable C-C double bond, as well as a further functional moiety that does not take part in the polymerization process. Preferably, this functional group is linked to the main polymer subchains of the polyfunctional polymer network via a C₂-C₁₀, more preferably a C₃-C₇ alkyl chain as a spacer.

The spacer molecules can be part of the functionalized monomers. Suitable monomers for this approach include acrylic and methacrylic esters or amides of C₂-C₁₀ alcohols or C₂-C₁₀ amines. In order to serve as spacers, these alcohols or amines carry an additional functional group at the terminal opposite to the one forming the ester or amide bond. This functional group either represents the one necessary for the interaction with the sample or probe molecules, or can be transformed to such a suitable functional group in a further step.

Alternatively, it is also possible to attach these spacer molecules to suitable reactive segments within the polymer subchains of the polyfunctional polymer network after its formation. In this case, reactive monomers have to be present during polymerization, such as acrylic or methacrylic acid chlorides or reactive esters thereof, as N-hydroxy succinimides or other monomers, e.g. maleic anhydride. These preferred reactive monomers can form covalent bonds to the bifunctional alcohols or amines that may be used as spacers.

The monomers carrying the spacer unit can readily be synthesized from the respective acrylic or methacrylic acid chloride or anhydride and the ω-amino or hydroxy carboxylic acid. The resulting product can be transformed to the active ester derivative by using e.g. N-hydroxy succinimide. A detailed procedure for the synthesis of several examples of such monomers can be found in the literature, e.g. in H.-G. Batz, J. Koldehoff, Macromol. Chem. 177 (1976) 683.

As outlined above, it is possible to use reactive monomers which directly yield the polymer subchains of the polyfunctional polymer network. Alternatively, monomers can be chosen which carry a precursor of the functional group to be used on the final surface, e.g. an acid chloride or an acid anhydride. They can subsequently be transformed to reactive groups, e.g. NHS ester or glycidylester groups, which allow an interaction of the polyfunctional polymer network with sample or probe molecules under the desired conditions.

Thus, all polymerizable monomers are suitable for the purposes of the present invention, as long as they can be combined with, or comprise, functional groups necessary to allow an interaction of the polyfunctional polymer network with the sample molecules or probe molecules.

Functional groups which can be used for the purposes of the present invention are preferably chosen according to the molecules with which an interaction is to be achieved. The interaction can be directed to one single type of sample molecule, or to a variety of sample molecules. Since one important application of the present invention is the detection of specific molecules in biological samples, the functional groups present within the polyfunctional polymer network will preferably interact with natural or synthetic biomolecules which are capable of specifically interacting with the molecules in biological samples, leading to their detection. Suitable functional moieties will preferably be able to react with nucleic acids and derivatives thereof, such as DNA, RNA or PNA, e.g. oligonucleotides or aptamers, polysaccharides, proteins including glycosidically modified proteins or antibodies, enzymes, cytokines, chemokines, peptide hormones or antibiotics or peptides or labeled derivatives thereof.

Moreover, it will be possible to conduct the coupling reaction between the molecules to be detected or the synthetic oligonucleotides and the polyfunctional polymer network under conditions which are not detrimental to the sample or probe molecules. Consequently, in an nucleic acid sensor application, the reaction should be carried out in an aqueous solution, and the temperature should not be raised above 95°C.

Also, the coupling reaction should proceed at a reasonable rate so that the detection can preferably be accomplished within less than 24 hours without requiring extreme pH-values in the solution. For the immobilization of synthetic oligonucleotide single strands, the pH should range between 7 and 11, preferably 7 to 10. During the hybridization reaction of the nucleic acid sample molecules with the probe molecules, the bond between the functional group and the synthetic oligonucleotide single strand as well as the fixing of the polyfunctional polymer network to the substrate have to be able to withstand temperatures of more than 65 °C, and a pH of 6-9. In cases where DNA is used as a sample molecule, the temperatures may have to be raised up to about 95°C in order to effect a separation of the DNA strands, which is necessary for hybridization.

Since most of the probe molecules, especially in biological or medical applications, comprise sterically unhindered nucleophilic moieties, preferred interactions with the polyfunctional polymer network comprise nucleophilic substitution or addition reactions leading to a covalent bond between the polymer subchains and the sample or probe molecules. For example, synthetical oligonucleotides are usually provided with a free amine group at one end (5'or 3'). Thus, exemplary functional groups provide, for example, a reactive double bond, an equivalent for a double bond (as e.g. an epoxy group) or a reactive leaving group. However, ionic or vander-Waals forces as well as hydrogen bonds can also be used to couple sample molecules to the polyfunctional polymer network if the functional groups are chosen accordingly.

Preferred functional groups can be chosen from prior literature with respect to the classes of molecules which are to be immobilized and according to the other requirements (reaction time, temperature, pH value) as described above. A general list can for example be found in the text book "Bioconjugate Techniques" by G. T. Hermanson, Academic Press, 1996. In the case of the attachment of amino-terminated oligonucleotides, examples for suitable groups are so-called active or reactive esters as N-hydroxy succinimides (NHS-esters), epoxides, preferably glycidyl derivatives, isothiocyanates, isocyanates, azides, carboxylic acid groups or maleinimides.

As preferred functional monomers which directly result in polyfunctional polymer subchains of the polyfunctional polymer network, the following compounds can be employed for the purposes of the present invention:
- acrylic or methacrylic acid N-hydroxysuccinimides,
- N-methacryloyl-6-aminopropanoic acid hydroxysuccinimide ester,
- N-methacryloyl-6-aminocapronic acid hydroxysuccinimide ester or
- acrylic or methacryl acid glycidyl esters.

Depending on the application, there is the possibility of providing the polymer subchains of the polyfunctional polymer network with a combination of two or more different functional groups, e.g. by carrying out the polymerization leading to the polymer subchains in the presence of different types of functionalized monomers. Alternatively, the functional groups may be identical.

The polymer subchains of the polyfunctional polymer network may for example be prepared via a chain reaction and may be cross-linked simultaneously. While radical mechanisms are preferred for practical reasons, the application of ionic or other polymerization techniques is also possible.

If a thermally initiated radical mechanism is used, for example peroxo groups or azo groups containing polymerization initiators may be used. Aromatic ketones such as benzoin, benzil or benzophenone derivatives may be preferably used as polymerization initiators if the polymers are formed by photochemical initiation. Aromatic ketones comprising sulphur may equally be used, if desired, in order to shift the suitable wavelength for photoinitiation to a longer wavelength region. As an example there may be mentioned aromatic β-keto sulfides, aromatic β-keto sulfoxides or aromatic β-keto sulfones. It is appreciated that the same or different initiators may be used in the polymerization mixture.

The functional groups comprised in the bifunctional linkers used in one embodiment of the inventive method for surface fixing have to be adapted to the surface used. For metal oxides, especially silicon oxide surfaces (evaporated or sputtered SiOₓ layers, SiO₂ surfaces of silicon wafers, glass, quartz), chlorosilane moieties or alkoxysilanes may be used. Thiol or disulfide groups can be employed for fixing to gold surfaces. Silanes are usually preferred due to their increased stability on surfaces. However, the method of the present invention - and this is a remarkable advantage - is not restricted to inorganic surfaces. Any surface, particularly desirable organic polymer surfaces, can be used as substrate to carry the polyfunctional polymer network. Examples for suitable organic polymers are cycloolefin copolymers (COCs), poly(methyl methacrylate) (PMMA, Plexiglass), polystyrene, polyethylene or polypropylene. A suitable COC is for example available from Ticona under the trade name "Topas".

Preferred examples for suitable cross-linkers are: bisacrylates, bismethacrylates, for example oligo-ethylene glycol bismethacrylates such as ethylene glycol bismethacrylate, and bisacrylamides, for example ethylene diamine bisacrylamide.

Upon initiation of the polymerization reaction, preferably by a heating step (thermal initiation) or exposure to radiation (photoinitiation) in the presence of polymerizable functionalized monomers and cross-linkers, polymer subchains can be formed and are simultaneously cross-linked. The polymerization can be carried out under standard reaction conditions known in the art.

Since it is possible to precisely control such parameters as cross-link density and swelling behavior, it is possible to adapt the properties of the respective polyfunctional polymer network to a variety of applications. By adjusting the reaction conditions networks with thicknesses ranging from a few nanometers up to some millimeters or even more may be prepared. It is also possible to fine-tune the properties of the resulting polyfunctional polymer network, e.g. with respect to the accessibility of the functional groups for subsequently coupled probe and sample molecules which may vary considerably in their size and structure.

Care should be taken to remove unreacted monomers as well as non-bonded or cross-linked polymer chains with suitable solvents after polymerization.

According to an alternative method, in a first step long polymer chains with appropriate functional groups are synthesized followed by cross-linking the latter.

Creating patterned arrays of the polyfunctional polymer network is possible by various means. One way are standard photolithographic processes that can either be applied after polymerization (photoablation of the polymers through masks) prior to this step (photodecomposition or photoablation of the initiator monolayer masks) or during the polymerization by means of photopolymerization through masks. Other possible techniques for the creation of patterned polyfunctional polymer networks are microcontact printing or related methods, which may be applied during polymerization. Finally, ink jet techniques or other microplotting methods can be used to create patterned polyfunctional polymer networks. Using any of these techniques, surface structures with dimensions in the micrometer range can be created. The high parallel mode of signal generation and a significant improvement in the integration of analytical data is the most promising feature of such techniques, which accordingly allow the optimization of automatic analytical procedures.

For the detection of a successful immobilization of sample or probe molecules on a polyfunctional polymer network, a variety of techniques can be applied. In particular, it has been found that the polyfunctional polymer networks undergo a significant increase in their thickness which can be detected with suitable methods, e.g. ellipsometry. Mass sensitive methods may also be applied.

If nucleic acids, for example oligonucleotides with a desired nucleotide sequence or DNA molecules in a biological sample, are to be analyzed, synthetic oligonucleotide single strands can be reacted with the polyfunctional polymer network. The reaction is carried out under high humidity, preferably in a buffered aqueous solution. The reaction temperature can be raised above room temperature, as long as it is not detrimental to the oligonucleotides. Preferred temperatures are in the range of 40-60°C. In this application, a multitude of identical synthetic oligonucleotide strands or a mixture of different strands can be used. If different strands are used, their sequences should preferably be known.

Before the thus prepared surface is used in a hybridization reaction, unreacted functional groups are deactivated via addition of suitable nucleophiles, preferably C₁-C₄ amines, such as simple primary alkylamines (e.g. propyl or butyl amine), secondary amines (diethylamine) or amino acids (glycin).

Upon exposure to a mixture of oligonucleotide single strands, e.g. as obtained from PCR, which are labeled, only those surface areas which provide synthetic strands as probes complementary to the PCR product will show a detectable signal upon scanning due to hybridization. In order to facilitate the parallel detection of different oligonucleotide sequences, printing techniques can be used which allow the separation of the sensor surface into areas where different types of synthetic oligonucleotide probes are presented to the test solution.

The term "hybridization" as used herein may relate to stringent or non-stringent conditions. If not further specified, the conditions are preferably non-stringent. Said hybridization conditions may be established according to conventional protocols described, for example, in Sambrook, "Molecular Cloning, A Laboratory Manual", Cold Spring Harbor Laboratory, N. Y. (1989), Ausubel, "Current Protocols in Molecular Biology", Green Publishing Associates and Wiley Interscience, N. Y. (1989), or Higgins and Hames (Eds) "Nucleic acid hybridization, a practical approach" IRL Press Oxford, Washington DC, (1985). The setting of conditions is well within the skill of the artisan and to be determined according to protocols described in the art. Thus, the detection of only specifically hybridizing sequences will usually require stringent hybridization and washing conditions such as for example 0.1 x SSC, 0.1% SDS at 65°C. Exemplary non-stringent hybridization conditions for the detection of homologous or not exactly complementary sequences may be set at 6 x SSC, 1% SDS at 65°C. As is well known, the length of the probe and the composition of the nucleic acid to be determined constitute further parameters of the hybridization conditions.

The nucleic acids to be analyzed may originate from a DNA library or a genomic library, including synthetic and semisynthetic nucleic acid libraries. Preferably, the nucleic acid library comprises oligonucleotides.

In order to facilitate their detection in an immobilized state, the nucleic acid molecules should preferably be labeled. Suitable labels include radioactive, fluorescent, phosphorescent, bioluminescent or chemoluminescent labels, an enzyme, an antibody or a functional fragment or functional derivative thereof, biotin, avidin or streptavidin.

Antibodies may include, but are not limited to, polyclonal, monoclonal, chimeric or single-chain antibodies or functional fragments or derivatives of such antibodies.

The general methodology for producing antibodies is well-known and has been described in, for example, Köhler and Milstein, Nature 256 (1975), 494 and reviewed in J. G. R. Hurrel, ed., "Monoclonal Hybridoma Antibodies: Techniques and Applications", CRC Press Inc., Boco Raron, FL (1982). Also the method taught by L. T. Mimms et al., Virology 176 (1990), 604-619, is applicable. As stated above the term "antibody" herein relates to monoclonal or polyclonal antibodies. Functional antibody fragments or derivatives provide the same specificity as the original antibody and comprise F(ab')₂, Fab, Fv or scFv fragments; see, for example, Harlow and Lane, "Antibodies, A Laboratory Manual", CSH Press 1988, Cold Spring Harbor, N. Y. Preferably the antibody used here is a monoclonal antibody. Furthermore, the derivatives can be produced by peptidomimetics. Such production methods are well known in the art and can be applied by the person skilled in the art without further ado.

Depending on the labeling method applied, the detection can be effected by methods known in the art, e.g. via laser scanning or use of CCD cameras.

Also applicable are methods where detection is indirectly effected. An example of such an indirect detection is the use of a secondary labeled antibody directed to a first compound such as an antibody which binds to the biological molecule (sample molecule) of interest.

A regeneration of the sensor surfaces after the immobilization has taken place is possible, but single uses are preferred in order to ensure the quality of results.

With the polyfunctional polymer networks different types of samples can be analyzed with an increased precision and/or reduced need of space in serial as well as parallel detection methods. The sensor surfaces to which the polyfunctional polymer networks have been fixed by the method according to the invention can therefore serve in diagnostical instruments or other medical applications, e.g. for the detection of components in physiological fluids, such as blood, serum, sputum etc.

The disclosure content of the documents cited throughout the specification are herewith incorporated by reference.

The embodiments of the present invention are further illustrated in the following items:

A preferred process for the detection of sample nucleic acid molecules, preferably of single stranded nucleic acid molecules, using a patterned active surface for bioconjugation produced by the method of the invention comprises the steps of:
allowing a hybridization reaction to take place between the oligonucleotide single strands forming the patterned array of the self-supporting film and the sample nucleic acid molecules,
removing the non-hybridized nucleic acid molecules in a washing step, and
detecting the hybridized nucleic acid molecules, preferably fluorometrically.

In the following the invention is disclosed in more detail with reference to examples. However, the described specific forms or preferred embodiments are to be considered in all respects as illustrative and not restrictive, the scope of the invention being indicated by the appended claims rather than by the following description, and all changes which come within the meaning and range of equivalency of the claims are therefore intended to be embraced therein.

### Examples

### Synthesis of a functionalized monomer

As an example, the synthesis of N-methacryloyl-6-aminocapronic acid hydroxysuccinimide ester is described. The reaction pathway is shown below. The indices i-iii in this figure refer to the description of the various steps in the text. 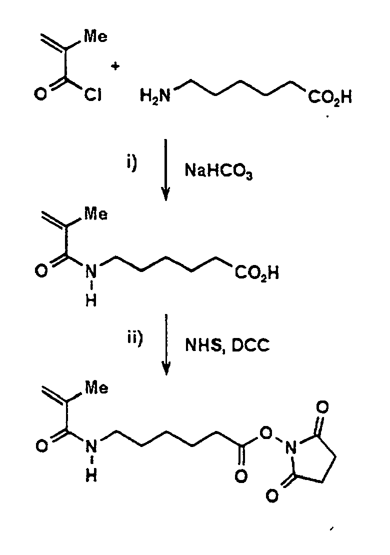
i) A solution of 13.2 g 6-aminocaproic acid and 20 g NaHCO₃ in 100 ml water and 50 ml 1,4-dioxane was slowly added to a solution of 10.3 ml of methacrylic acid chloride in 50 ml 1,4-dioxane. The solution was stirred overnight. Then 50 ml of water were added and the mixture was washed three times with 100 ml portions of ethyl acetate. The water layer was acidified (pH 2) with dilute hydrochloric acid and then extracted with three 100 ml portions of ethyl acetate. The combined organic layers were dried over Na₂SO₄, concentrated to a volume of about 50 ml and added to 350 ml of cold hexane. This mixture was cooled to -20°C and the product slowly separated overnight as white crystals (yield: ca. 14 g).
ii) A solution of 14 g of the acid in 300 ml methylene chloride was cooled to 5°C and 8.2 g of N-hydroxy succinimide (NHS) and 14.6 g of N,N-dicyclohexyl carbodiimide were added. The mixture was kept at 5°C overnight. The precipitate (dicyclohexylurea) was filtered off and the solvent was evaporated. During this step, additional urea separated in some cases and was also filtered off. The crude product was recrystallized from isopropanol to yield about 15 g of the NHS ester monomer.

### Formation of a polyfunctional polymer network

A solution comprising the following ingredients was used:
40 mole % N,N-dimethyl acrylamide (for the water-swellable basis polymer),
10 mole % N-methacryloyl-6-aminocapronic acid hydroxysuccinimide ester (for the functionalized repeating units),
5 mole % ethylene glycol bismethacrylate (for the cross-linking units),
1 mole % azobisisobutyronitril (as an initiator),
balance (to 100 mole %) ethanol.

After heating to 70°C polymerization was performed for 10 hours. Thereafter, the obtained polymer network was washed with ethanol and transferred onto a microscope slide covered with a layer of a benzophenone based bifunctional silane linker and fixed to the surface by irradiation with ultraviolet irradiation (500 watt Hg lamp from Oriel, 5 to 45 min irradiation time, with a dichroic filter at 320 to 420 nm or a cut-off filter at 320 nm).

### Detection of oligonucleotides strands

The obtained surface was exposed to 1 nl of a 10 µM oligonucleotide solution and the coupling reaction was allowed to proceed at about 40-50°C for two hours in an aqueous solution.

The synthetic oligonucleotide was 5-amino modified and the solution was buffered with a 100 mM sodium phosphate buffer at a pH of 8.0. After the coupling reaction, the sensor surface was rinsed with the sodium phosphate buffer. In order to define the spatial extension of the specific types of oligonucleotide on the sensor surface for parallel detection, the reactant was printed onto the polyfunctional polymer network.

The surface thus prepared was allowed to react with a Cy5 labeled PCR product in a buffer of 2 x SSC, 10% dextrane sulphate and 50% formamide for 12h at 28°C. The DNA content was 100 ng DNA/80 µl sample. After the hybridization reaction has taken place, the surface was washed in SSC-buffer and the result was detected fluorometrically via laser activation with a CCD camera. A fluorescence signal could only be detected for those areas which carried synthetic oligonucleotides complementary with the PCR product.

## Claims

1. Method for the large scale production of patterned active surfaces for bioconjugation comprising the steps of:
(a) preparing a self-supporting film of a polyfunctional polymer network comprising an assembly of cross-linked polymer subchains, wherein each polymer subchain comprises a multitude of identical of different repeating units carrying one or more functional groups which allow an interaction of the polymer with one or more probe molecules,
(b) providing said self-supporting film with patterned arrays of said one or more probe molecules via an interaction with said functional groups,
(c) fixing said self-supporting film on a solid surface, and
(d) wherein said functional groups of said polyfunctional polymer network are selected from the group consisting of carboxylic acids, maleinimides, N-hydroxy succinimides, epoxides, isothiocyanates, isocyanates, azides, NHS ester, glycidylester, acrylic or methacrylic acid N-hydroxysuccinimies, N-methacryloyl-6-aminopropanoic acid hydroxysuccinimide ester, N-methacryloyl-6-aminocapronic acid hydroxysuccinimide ester or acrylic or methacryl acid glycidyl esters.

2. Method according to claim 1, wherein said polymer subchains comprise segments that make said polymer network water-swellable.

3. Method according to claim 2, wherein said water-swellability is provided by monomers selected from the group consisting of acrylic acid, methacrylic acid, dimethyl acrylamide and vinyl pyrrolidone.

4. Method according to any one of the preceding claims, wherein for preparing said cross-linked polymer subchains a cross-linker selected from the group consisting of bisacrylates, bismethacrylates and bisacrylamides is used.

5. Method according to any one of the preceding claims, wherein each of said probe molecules is a partner of a specifically interacting system of complementary binding partners.

6. Method according to claim 5, wherein said specifically interacting system of complementary binding partners is based on nucleic acid/complementary nucleic acid, peptide nucleic acid/nucleic acid, enzyme/substrate, receptor/effector, lectin/sugar, antibody/antigen, avidin/biotin or streptavidin/biotin interaction.

7. Method according to any one of the preceding claims, wherein in step (c) said fixing to said solid surface is performed by using a reactive glue or a bifunctional linker system which comprises one or more functional groups suitable for covalently binding said linker system to said solid surface and one or more functional groups for covalently binding said polyfunctional polymer network to said covalently bound linker system.

8. Method according to claim 7, wherein said linker system comprises a halogen silane, an alkoxy silane, an acyloxy silane, an amino silane, a disulphide or a thiol group.

9. Method according to claim 7, wherein said linker system comprises a photoreactive group.

10. Method according to claim 9, wherein said photoreactive group is selected from the group consisting of aromatic ketones and aromatic ketones containing sulphur.

11. Method according to claim 10, wherein said photoreactive group is selected from the group consisting of an anthrathione group or a derivative thereof, an anthraquinone group or a derivative thereof, a benzophenone group or a derivative thereof.

12. Method according to any one of the preceding claims, wherein said solid surface is selected from the group consisting of a metal or semimetal surface, a metal oxide or semimetal oxide surface, and a polymer surface.

13. Method according to any one of the preceding claims, wherein in step (a) said self-supporting film of a polyfunctional polymer network is formed on one surface of a carrier film and in step (c) said carrier film is fixed on said solid surface with the other surface.

14. The method of any one of the preceding claims, wherein in a further step following step (b) said self-supporting film is cut into sheets or an endless tape of a desired format, wherein said tape may further optionally be wind-up onto a drum.

15. Patterned active surface for bioconjugation obtained by a method according to any one of claims 1 to 15.

16. Patterned active surface according to claim 15, which is planar or non-planar.

17. Patterned active surface according to claim 16, which is planar and part of a sensor chip.

18. Medical or diagnostic instrument, comprising a patterned active surface according to any one of claims 15 to 17.

## Patentansprüche

1. Verfahren zur groß angelegten Herstellung von gemusterten aktiven Oberflächen zur Biokonjugation umfassend die Schritte:
(a) Herstellen eines selbsttragenden Films eines polyfunktionellen Polymernetzwerks, umfassend eine Anordnung von vernetzten Polymerunterketten, wobei jede Polymerunterkette eine Vielzahl an identischen von unterschiedlichen Wiederholungseinheiten umfasst, die eine oder mehrere funktionelle Gruppen tragen, die eine Wechselwirkung des Polymers mit einem oder mehreren Sondenmolekülen ermöglichen,
(b) Versehen des selbsttragenden Films mit gemusterten Arrays aus dem einen oder den mehreren Sondenmolekülen über eine Wechselwirkung mit den funktionellen Gruppen,
(c) Fixieren des selbsttragenden Films auf einer festen Oberfläche und
(d) wobei die funktionellen Gruppen des polyfunktionellen Polymernetzwerks ausgewählt sind aus der Gruppe bestehend aus Carbonsäuren, Maleinimiden, N-Hydroxysuccinimiden, Epoxiden, Isothiocyanaten, Isocyanaten, Aziden, NHS-Ester, Glycidylester, Acryl- oder Methacrylsäure-N-hydroxysuccinimiden, N-Methacryloyl-6-aminopropansäurehydroxysuccinimidester, N-Methacryloyl-6-aminocapronsäurehydroxysuccinimidester oder Acryl- oder Methacrylsäureglycidylestern.

2. Verfahren nach Anspruch 1, wobei die Polymerunterketten Segmente umfassen, die das Polymernetzwerk wasserquellbar machen.

3. Verfahren nach Anspruch 2, wobei die Wasserquellbarkeit durch Monomere bereitgestellt wird, ausgewählt aus der Gruppe bestehend aus Acrylsäure, Methacrylsäure, Dimethylacrylamid und Vinylpyrrolidon.

4. Verfahren nach einem der vorangehenden Ansprüche, wobei zum Herstellen der vernetzten Polymerunterketten ein Vernetzer, ausgewählt aus der Gruppe bestehend aus Bisacrylaten, Bismethacrylaten und Bisacrylamiden verwendet wird.

5. Verfahren nach einem der vorangehenden Ansprüche, wobei jedes der Sondenmoleküle ein Partner eines spezifisch wechselwirkenden Systems von komplementär bindenden Partnern ist.

6. Verfahren nach Anspruch 5, wobei das spezifisch wechselwirkende System von komplementär bindenden Partnern auf der Basis der Wechselwirkung von Nukleinsäure/komplementärer Nukleinsäure, Peptidnukleinsäure/Nukleinsäure, Enzym/Substrat, Rezeptor/Effektor, Lectin/Zucker, Antikörper/Antigen, Avidin/Biotin oder Streptavidin/Biotin erfolgt.

7. Verfahren nach einem der vorangehenden Ansprüche, wobei in Schritt (c) das Fixieren an der festen Oberfläche unter Verwendung eines reaktiven Klebstoffs oder eines bifunktionellen Linkersystems durchgeführt wird, das eine oder mehrere Gruppen, die zum kovalenten Binden des Linkersystems an der festen Oberfläche und eine oder mehrere funktionelle Gruppen zum kovalenten Binden des polyfunktionellen Polymernetzwerks an dem kovalent gebundenen Linkersystem geeignet sind, umfasst.

8. Verfahren nach Anspruch 7, wobei das Linkersystem ein Halogensilan, ein Alkoxysilan, ein Acyloxysilan, ein Aminosilan, ein Disulfid oder eine Thiolgruppe umfasst.

9. Verfahren nach Anspruch 7, wobei das Linkersystem eine photoreaktive Gruppe umfasst.

10. Verfahren nach Anspruch 9, wobei die photoreaktive Gruppe ausgewählt ist aus der Gruppe bestehend aus aromatischen Ketonen und schwefelhaltigen aromatischen Ketonen.

11. Verfahren nach Anspruch 10, wobei die photoreaktive Gruppe ausgewählt ist aus der Gruppe bestehend aus einer Anthrathiongruppe oder einem Derivat davon, einer Anthrahinongruppe oder einem Derivat davon, einer Benzophenongruppe oder einem Derivat davon.

12. Verfahren nach einem der vorangehenden Ansprüche, wobei die feste Oberfläche ausgewählt ist aus der Gruppe bestehend aus einer Metall- oder Halbmetalloberfläche, einer Metalloxid- oder Halbmetalloxidoberfläche und einer Polymeroberfläche.

13. Verfahren nach einem der vorangehenden Ansprüche, wobei in Schritt (a) der selbsttragende Film eines polyfunktionellen Polymernetzwerks auf einer Oberfläche eines Trägerfilms gebildet wird und in Schritt (c) der Trägerfilm auf der festen Oberfläche mit der anderen Oberfläche fixiert wird.

14. Verfahren nach einem der vorangehenden Ansprüche, wobei in einem weiteren Schritt nach Schritt (b) der selbsttragende Film in Lagen oder ein Endlosband mit einem gewünschten Format geschnitten wird, wobei das Band weiter wahlweise auf eine Trommel aufgewickelt werden kann.

15. Gemusterte aktive Oberfläche zur Biokonjugation, erhalten durch ein Verfahren gemäß einem der Ansprüche 1 bis 15.

16. Gemusterte aktive Oberfläche nach Anspruch 15, die eben oder nicht eben ist.

17. Gemusterte aktive Oberfläche nach Anspruch 16, die eben und ein Teil eines Sensorchips ist.

18. Medizinisches oder diagnostisches Instrument, umfassend eine gemusterte aktive Oberfläche nach einem der Ansprüche 15 bis 17.

## Revendications

1. Procédé de production à grande échelle de surfaces actives à motifs pour la bioconjugaison, comprenant les étapes consistant à :
(a) préparer un film autoporteur en un réseau polymère polyfonctionnel, comprenant un ensemble de sous-chaînes polymères réticulées, dont chaque sous-chaîne polymère comprend une multitude d'unités répétitives identiques ou différentes portant un ou plusieurs groupes fonctionnels qui permettent une interaction du polymère avec une ou plusieurs molécules d'essai,
(b) munir ce film autoporteur de systèmes à motifs d'une ou de plusieurs molécules d'essai au moyen d'une interaction avec les groupes fonctionnels,
(c) fixer le film autoporteur sur une surface solide, et
(d) choisir les groupes fonctionnels du réseau polymère polyfonctionnel dans le groupe composé des acides carboxyliques, des maléinimides, des N-hydroxysuccinimides, des époxydes, des isothiocyanates, des isocyanates, des azides, des esters de N-hydroxysuccinimides, des esters de glycidyles, des N-hydroxysuccinimides d'acide acrylique ou méthacrylique, des esters d'hydroxysuccinimides d'acide N-méthacryloyl-6-aminopropanoique, des esters d'hydroxysuccinimides d'acide N-méthacryloyl-6-aminocapronique ou des esters de glycidyle d'acide acrylique ou méthacrylique.

2. Procédé selon la revendication 1, selon lequel les sous-chaînes polymères comprennent des segments qui rendent ce réseau polymère gonflant à l'eau.

3. Procédé selon la revendication 2, selon lequel la capacité de gonfler à l'eau est fournie par des monomères choisis dans le groupe composé de l'acide acrylique, de l'acide méthacrylique, du diméthylacrylamide et de la vinylpyrrolidone.

4. Procédé selon l'une des revendications précédentes, selon lequel on utilise, pour préparer les sous-chaînes polymères réticulées, un réticulant choisi dans le groupe composé des bisacrylates, des bisméthacrylates et des bisacrylamides.

5. Procédé selon l'une des revendications précédentes, selon lequel chacune des molécules d'essai est un partenaire d'un système à interaction spécifique de partenaires à liaisons complémentaires.

6. Procédé selon la revendication 5, selon lequel le système à interaction spécifique de partenaires à liaisons complémentaires est basé sur l'interaction acide nucléique/acide nucléique complémentaire, acide peptide nucléique/acide nucléique, enzyme/substrat, récepteur/effecteur, lectine/sucre, anticorps/antigène, avidine/biotine ou streptavidine/biotine.

7. Procédé selon l'une des revendications précédentes, selon lequel à l'étape (c) la fixation à une surface solide est réalisée en utilisant une colle réactive ou un système lieur bifonctionnel qui comprend un ou plusieurs groupes fonctionnels convenant pour lier de façon covalente ce système lieur à la surface solide et un ou plusieurs groupes fonctionnels pour lier de façon covalente le réseau polymère polyfonctionnel au système lieur lié de façon covalente.

8. Procédé selon la revendication 7, selon lequel le système lieur comprend un groupe silane halogène, silane alcoxy, silane acyloxy, amino-silane, disulfure ou thiol.

9. Procédé selon la revendication 7, selon lequel le système lieur comprend un groupe photoréactif.

10. Procédé selon la revendication 9, selon lequel le groupe photoréactif est choisi dans le groupe composé de cétones aromatiques et de cétones aromatiques contenant du soufre.

11. Procédé selon la revendication 10, selon lequel le groupe photoréactif est choisi dans le groupe composé d'un groupe anthrathione ou d'un dérivé de celui-ci, d'un groupe anthraquinone ou d'un dérivé de celui-ci, d'un groupe benzophénone ou d'un dérivé de celui-ci.

12. Procédé selon l'une des revendications précédentes, selon lequel la surface solide est choisie dans le groupe composé d'une surface métallique ou semi-métallique, d'une surface en oxyde métallique ou oxyde semi-métallique et d'une surface en polymère.

13. Procédé selon l'une des revendications précédentes, selon lequel à l'étape (a) le film autoporteur en un réseau polymère polyfonctionnel est formé sur une surface d'un film porteur et à l'étape (c) le film porteur est fixé sur la surface solide avec l'autre surface.

14. Procédé selon l'une des revendications précédentes, selon lequel dans une autre étape consécutive à l'étape (b) le film autoporteur est découpé en feuilles ou en un ruban sans fin d'un format souhaité, ce ruban pouvant être également, en option, enroulé sur un tambour.

15. Surface active à motifs pour bioconjugaison obtenue par le procédé selon l'une quelconque des revendications 1 à 15.

16. Surface active à motifs selon la revendication 15, qui est plane ou non plane.

17. Surface active à motifs selon la revendication 16, qui est plane et fait partie d'une puce de détection.

18. Instrument médical ou de diagnostic, comprenant une surface active à motifs selon l'une quelconque des revendications 15 à 17.
